(19) Europäisches Patentamt
European Patent Office
Office européen des brevets

(11) Publication number: **0 336 779 A2**

# (12) EUROPEAN PATENT APPLICATION

(21) Application number: 89303469.4

(22) Date of filing: 07.04.89

(51) Int. Cl.[4]: **C 07 K 3/08**
C 07 K 1/00, C 07 K 5/04,
C 07 K 7/04

(30) Priority: 08.04.88 US 179160

(43) Date of publication of application:
11.10.89 Bulletin 89/41

(84) Designated Contracting States:
AT BE CH DE ES FR GB GR IT LI LU NL SE

(71) Applicant: SCRIPPS CLINIC AND RESEARCH FOUNDATION
10666 North Torrey Pines Road
La Jolla California 92037 (US)

(72) Inventor: Satterthwait, Arnold C., Jr.
13681 Ruette le Park
Del Mar California 92014 (US)

Arrhenius, Thomas
3909 San Augustine Way
San Diego California 92130 (US)

(74) Representative: Harrison, David Christopher et al
MEWBURN ELLIS & CO 2/3 Cursitor Street
London EC4A 1BQ (GB)

## (54) Polypeptides stabilized by covalent hydrogen bond replacements.

(57) Polypeptides are restricted to particular conformations in solution by replacing one or more hydrogen bonds with covalent hydrogen bond mimics of class 1 or class 2:

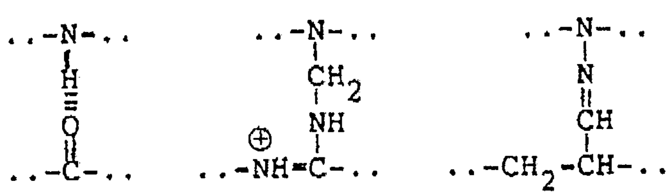

Normal class 1 class 2

The polypeptides thus stabilized are capable of greater biological activity.

EP 0 336 779 A2

**Description**

## POLYPEPTIDES STABILIZED BY COVALENT HYDROGEN BOND REPLACEMENTS

Technical Field

The invention relates to peptide chemistry and synthesis. In particular, it relates to assurance of three-dimensional conformation of peptides by replacement of one or more putative hydrogen bonds with a covalently bound surrogate.

Background Art

It has been well understood for decades that the three-dimensional conformation of a protein, as much as its primary amino acid sequence, is determinative of the effectiveness of the protein in its biological function. To some degree, of course, the three-dimensional conformation is an automatic consequence of the primary structure in the appropriate environment. However, a large component in the factors accounting for the three-dimensional conformation is the ability of the components of the amide linkages to form interchain and mostly intrachain hydrogen bonds. Occasionally, sidechain amides participate in hydrogen bond linkages, as well. The active regions of many proteins comprise relatively few amino acids. However, these amino acid sequences, when isolated from the rest of the protein, show little or no activity. This is because short polypeptides (e.g., <20 aa) are disordered in $H_2O$ and have no fixed structure. Nature orders these active sequences by incorporating them into proteins where collective forces induce a three-dimensional structure. Accordingly, attempts have been made to stabilize the appropriate three-dimensional conformation of short peptides using linkages that are resistant to disruption.

Three major approaches to such stabilization have been utilized. First, peptides have been cyclized by linking to N and C-termini in an amide bond to provide similar shapes to those of the corresponding native proteins. C.M. Deber et al, Acct Chem Res (1976) 9:106; D.F. Veber et al, Nature (1981) 282:55. This approach is most effective with relatively small peptides, such as somatostatin. In the Veber paper, for example, a bicyclic analog was shown to have a potency equal or greater than that of somatostatin.

In a second approach, disulfide bridges are formed in vitro to stabilize conformation. See, for example, A. Ravi et al, Tetrahedron (1984) 40:2577; R. Kishore et al, J Am Chem Soc (1985) 107:2986. These papers concern stabilization of beta-turn conformations in a series of cyclic peptides, and beta-sheets in cyclic peptides, respectively.

Finally, turn structures have been rigidified by utilizing modified amino acid side chains in cyclization reactions. See, for example, R.M. Freidinger et al, Science (1980) 210:656; J.L. Krstenansky et al, Biochem Biophys Res Commun (1982) 109:1368; D.S. Kemp et al, Tetrahedron Lett (1982) 23:3759; D.S. Kemp et al, Tetrahedron Lett (1982) 23:3761; U. Nagai et al, Tetrahedron Lett (1985) 26:647; M. Feigel et al, J M Chem Soc (1986) 108:181; M. Kahn et al, Tetrahedron Lett (1986) 27:4841. For example, the Nagai paper describes the synthesis of a bicyclic amino acid which had an almost superimposable conformation on that of D-Ala-L-Pro of a type II beta-turn in (D-Ala)-gramicidin. The Freidinger paper describes the synthesis of an LHRH analog using a lactam conformation constraint in the component amino acids.

T. Arrhenius et al, in a preview of the below-described work, presented at the UCLA/DuPont Protein Structure Meeting in April 1987 a poster which suggested the use of covalent substitutes for hydrogen bonding in helices, beta sheets, and turns, and described a hydrazone linkage substitute for an $i \longrightarrow i+4$ hydrogen bond. This appears to be the first suggestion that hydrogen bonding can be covalently replaced by alternative covalent linkages. This and analogous substitutions would have the advantage of providing a reliable and easily used method for assuring conformation in a wide variety of peptides.

Disclosure of the Invention

The invention herein provides a series of moieties which can be used in place of amino acids participating in conformation-stabilizing hydrogen bonds to achieve stability of the desired three-dimensional conformations. The invention also provides methods for substituting the moieties for amino acids. The method is most readily useful in stabilizing peptides whose three-dimensional shape depends on the integrity of the hydrogen bonds, however the linkages provided by the invention could also be provided in other locations where their presence is consistent with the desired shape.

Thus, in one aspect, the invention relates to peptides whose conformation is stabilized by replacement of at least one hydrogen bond (and the participating amino acids) with a covalently linked moiety which forms a spatially equivalent linkage. The invention is directed to these peptides, to methods to produce them, and to methods to utilize such peptides by substituting them for native proteins. Frequently, one covalent hydrogen bond mimic is sufficient to restrict the polypeptide to an active conformation. For example, once a single turn of a helix is established, any additional amino acid extension to the chain will tend to form additional helical turns. Thus the compounds provided in Schemes 2, 3 and 5 are directly useful for stabilizing helices and reverse turn conformations in polypeptides derived therefrom.

The hydrogen bond mimics may be used to improve the biological activity of any peptide and will find use in replacing hormones and in synthetic vaccines.

For example, an active fragment of epidermal growth factor (EGF), comprising amino acids 20-31, has been

identified by A. Komoriya et al, Proc Nat Acad Sci USA, 81:1351-55 (1984). The peptide's activity is about $10^{-4}$ that of the parent molecule. In the intact protein, residues 20-23 and 28-31 form anti-parallel beta sheets, joined by residues 24-27 in a reverse turn (G.T. Montellione et al, Proc Nat Acad Sci USA, 83:8594-98 (1986)). Either class of mimics of the present invention may be used to stabilize the conformation of the peptide fragment. For example, a class 1 aminoethaneamidinium bridge (Scheme 3) may be employed between residues 24 and 27 to stabilize the reverse turn structure. A class 2 bridge may be used to stabilize the T30(N-H):M21(C=O) hydrogen bond and/or the N32(N-H):V19(C=O) hydrogen bond. ("T30", "M21", etc., indicate the amino acid in single letter code and its position in the intact protein. Thus, T30 is the threonine at position 30.) Thus, one could prepare a series of EGF peptide derivatives of increasing size. Alternatively, one may use the smallest stabilized structure (the class 1 example) and add the appropriate amino acid sequences using conventional peptide chemistry.

Covalent mimics may also be employed to conformationally restrict synthetic peptide vaccines. Peptides shaped to correspond to the three-dimensional surface of proteins should induce antibodies with binding pockets which closely complement the native protein surfaces. Three-dimensional complementarity between the antibody and protein surfaces should lead to tighter binding interactions and improve the affinity. The overall immune response to conformationally restricted peptides should be greater than to unrestricted peptides of the prior art.

## Brief Description of the Drawing

Figure 1 depicts a hydrogen bond between two polypeptide chains, or two portions of one polypeptide chain ("normal"). This is contrasted with the mimic of class 1, in which the hydrogen bond and carbonyl groups are replaced with a methylene amidine, and a mimic of class 2, in which the hydrogen bond and carbonyl group are replaced by an imide.

## Modes of Carrying Out the Invention

### A. Definitions

As used herein, "protein" and "polypeptide" and "peptide" are used interchangeably to designated polymers or oligomers formed by amide linkages of amino acids. These terms are used interchangeably, regardless of the number of amino acid residues in the resulting structure. These terms are also used to denote structures which are fundamentally polymeric amino acids, even though one or more of said "amino acids" may be slightly modified to accommodate the linking moieties of the invention, or may be an amino acid analog. Unless otherwise noted, the amino acid residues are in the L configuration, but included within the invention are proteins which contain residues of the D configuration, as well as protein mixtures of enantiomeric forms of one or more amino acids. Additionally, one may include amino acids modified by substitution on the alpha carbon and/or amide nitrogen. Suitable substitutions include, for example, halo, alkyl of 1-6 carbons, acyl of 1-6 carbons, benzyl, haloalkyl of 1-6 carbons, and the like.

The term "polypeptide analog" as used herein refers to compounds of the invention, wherein one or more natural amino acids are replaced by amino acyl moieties bearing one of the covalent linkers of the invention. A polypeptide analog has the same nominal amino acid sequence as the segment of the protein it mimics, and differs by the covalent radical replacement of stabilizing hydrogen bonds.

"Hydrogen bond" is used in the conventional sense to designate the relatively weak, noncovalent interaction between a hydrogen covalently bonded to carbon or to an electronegative atom, such as nitrogen, oxygen, or sulfur, with an unshared electron pair of an electron donor atom, such as O, N, or S. The geometry of the hydrogen bond approaches linearity between the electron donating atom, the hydrogen, and the atom to which it is attached; however, it is understood that a reasonable deviation from linearity is included, and, indeed, usually found.

By "corresponding native peptide" is meant the protein which the peptide of the invention is intended to replace. Thus, for example, analogs of LHRH, somatostatin, growth hormones, enzymes such as the various serine proteases, antiviral proteins such as the interferons, lymphokines such as TNF, lymphotoxin, and the colony stimulating factors, immunodominant epitopes found on the surface of infective pathogens, and so forth are generally assured of their three-dimensional conformations by virtue of hydrogen bonding at various locations by formation of intrachain and interchain linkages. Replacement of these hydrogen bonds by the spatially equivalent covalent linkage prepared by the method of the invention results in a peptide corresponding to these native proteins.

By "spatially equivalent covalent linkage" is meant a substitute for the hydrogen bonding participants which contains only covalent bonds, and which holds the chains in essentially the same conformation as they would have been held by the hydrogen bonds. "Spatially equivalent" is also understood to accommodate minor deviations in exact spatial replication, so long as the activity of the resultant peptide is maintained. For the purposes of the instant invention, "spatially equivalent covalent linkages" include $-(N)-CH_2-NH-(C)-$, $-(N)-CH_2CH_2-NH-(C)-$, and $-(N)-N=CH-(C)-$, where the atoms in parentheses donote amide and carbonyl peptide backbone atoms. (Even though $-CH_2CH_2NH-$ is not strictly equivalent in length to a hydrogen bond, we have found it quite useful in stabilizing certain structures, e.g., reverse turns.)

B. Survey of Conformations and Hydrogen Bond Types

The results of a reasonable number of x-ray crystallographic studies of protein conformations have permitted the assessment of shapes and the assignment of the nature of hydrogen bonds securing these conformations at a molecular level. Certain conventions have been followed in designating the types of hydrogen bonds denoted. In general, the participants are designated in terms of a reference residue, i, and an additional residue (i + #) which is that residue which is located the designated number (#) of residues further downstream (starting from the C-terminus of the chain). The hydrogen bond itself is designated by an arrow which points in the direction of hydrogen donation--i.e., the arrow points from the residue donating the hydrogen toward the residue bearing the electrons that accepts them. Thus, for example, an i + 4 ---→ i hydrogen bond is the donation of the amide by hydrogen from the i + 4 residue to the oxygen of the carbonyl group of the i residue is 4 amino acids downstream. A hydrogen bond between these participants results in an alpha helix.

Table 1 shows the types of hydrogen bonds which are recognized to form commonly encountered conformational characteristics of single-chain- and interchain-linked proteins. Some of these conformations are more frequently encountered than others. Approximately 60-70% of all protein is found in the form of either alpha helices, beta sheets, or beta turns. Other forms of turns and helices are also found, but are less common. In Table 1, the type of hydrogen bond is shown along with the ring size--i.e., the number of atoms forming the resulting ring (including the electron-donating atom and the hydrogen). A detailed description of these structures is provided by G. Nemethy & H.A. Scherage, BBRC, 95:320 (1980). The resulting structure is shown as the type of secondary or tertiary structure obtained as a consequence of the hydrogen bond type given.

Table 1

| Hydrogen Bond | Ring Size | Secondary or Tertiary Structure |
|---|---|---|
| i + 2 ---→ i | 7 | $2_7$ ribbon, Type $II_3$,$II_1$, reverse turns |
| i + 3 ---→ i + l | 7 | Type I,$II_3$ reverse turns |
| i + 3 ---→ i | 10 | $3_{10}$ helix, Type I,$II_1$,IV reverse turns |
| i + 4 ---→ i | 13 | alpha helix, |
| i ---→ i + 4 | 14 | type IV reverse turn antiparallel beta-sheet (across a Type 1 or Type II turn) |
| i + 5 ---→ i | 16 | pi helix |
| i + 7 ---→ i | 22 | antiparallel beta-sheet (across a turn) |

In addition to these intrachain linkages, antiparallel and parallel beta-sheet hydrogen bonding can also be substituted by the covalent linkers of the invention. The schematics of these interchain bonds are as follows:

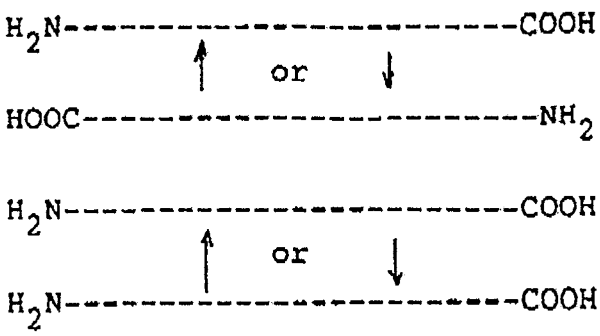


antiparallel

parallel

In addition to the foregoing, since asparagine, glutamine, serine and cysteine donate or accept hydrogen atoms, sidechain-sidechain and sidechain-backbone hydrogen bonds can be formed, for example, between two asparagine residues, asparagine and glutamine, or glutamine and glutamine, or between these amide sidechains and the backbone residues.

The covalent moieties which mimic the hydrogen bonds can be used to replace any of the foregoing linkages to maintain the desired secondary or tertiary structure.

C. Types of Hydrogen Bond Mimics

In general, two classes of mimics have been found which are successful in replicating the geometry of the hydrogen bonds of native proteins. These are shown in comparison to the typical hydrogen bonds in Figure 1.

As shown in Figure 1, the first class comprises an N-methylamino amidine link, when X is N. In this embodiment, a methylene or ethylene group replaces the hydrogen attached to the alpha-amino group of the i + # amino acid and the electron-donating oxygen of the carboxyl group of the i amino acid is replaced by nitrogen.

The second class of covalent linkers are hydrazone-hydrocarbon links. In this embodiment, the hydrogen of the alpha amino of the i + # amino acid is replaced by a nitrogen, which is, in turn, covalently linked by a double bond to a carbon which takes the place of the carboxyl oxygen of the i residue. In this embodiment, the alpha-amino group of the i + 1 residue may be replaced by carbon, and linked to residue i through a single or double covalent bond.

Either of the foregoing classes of mimics can be used in the hydrogen bond configurations shown in Table 1 and in interchain bonds. The synthesis of these mimics in the context of peptide chains is illustrated below. When synthesized, the mimics may be chain-extended to form the remainder of the peptides using standard protein synthesis techniques.

Synthesis of Class 1 Mimics

The basic reaction for formation of class 1 linkers can be conducted using a thioamide and a protected or unprotected primary amine so as to displace the methylthio substituent according to the reaction:

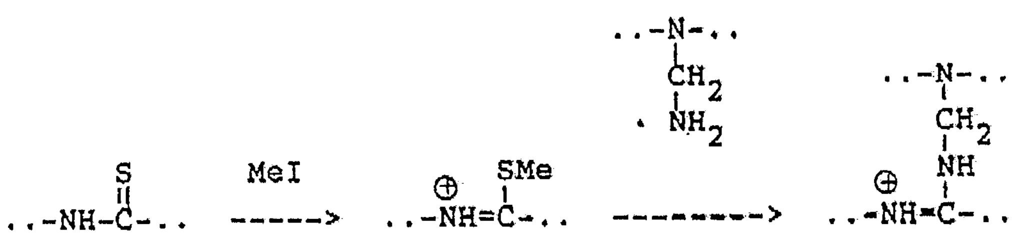


In the foregoing reaction, "..-" indicates extension of the peptide chain.

Reaction scheme 1 shows the application of this reaction to the formation of the desired linkage in the context of the intrachain mimic:

Scheme 1

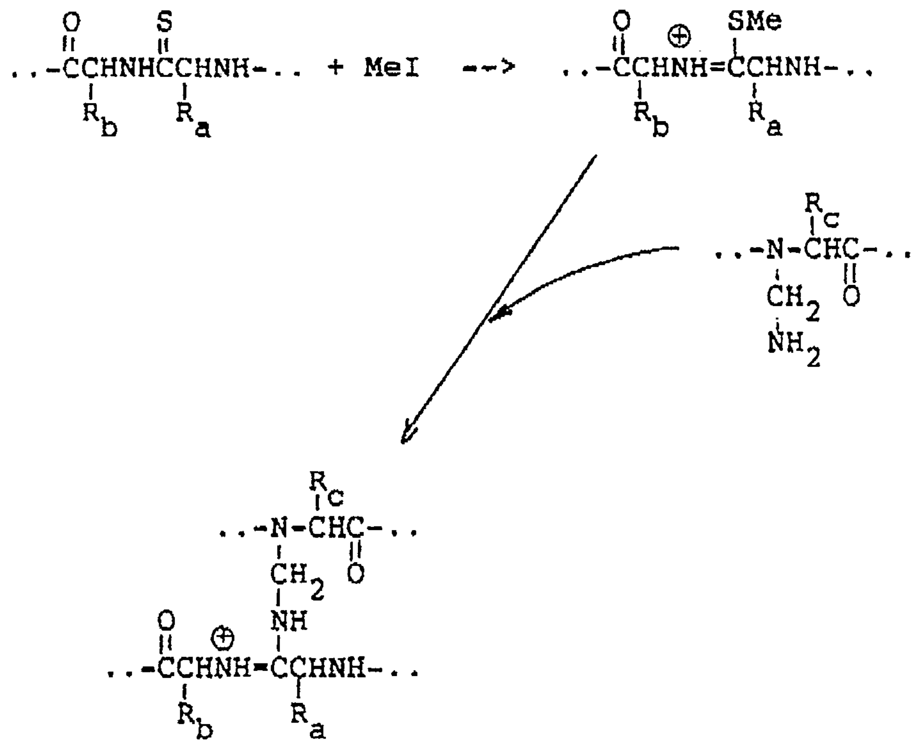


For adaptation to peptide synthesis, the carboxyl terminus of the protein (beyond the $R_c$-bearing residue) is bonded to a resin, and intermediate amino acids and analogues are included to complete the intervening chain. For example, for an i ---→ i + 4 alpha helix-forming mimic, a tripeptide sequence is placed intermediate to the residue containing the aminomethyl substituent and the residue containing the thioamide, according to reaction scheme 2.

The basic reaction is the displacement of a methylthio substituent using a primary amine which is itself a methylamino substituent to an amide nitrogen of the same or different chain in the peptide. According to the schematic used to illustrate the invention herein, the oxygen of the carbonyl which would have been the electron donor atom in the hydrogen bond is replaced by a sulfur, methylated, and then displaced using the amino methyl substituent which replaces the hydrogen which would have ordinarily accepted the electron donation. As shown in reaction scheme 1, the resulting amino methylene linkage replaces the donated electrons and the recipient hydrogen.

The detailed chemistry for introducing a class 1 mimic into a peptide chain was developed in the context of replacing an (1+4) —→ i hydrogen bond in one turn of an alpha helix, and is depicted in Scheme 2. The reactions for each step are set forth in the Examples below. The structure of the final product is verified using nmr. Model studies show that the final product should form one turn of an alpha-helix. Approximately 25% of native protein exhibit helical conformations.

In the Schemes below, the following abreviations are used: Cbz = carbobenzoxy, Me = methyl, Et = ethyl, Bu = butyl, Ac = acetyl ($CH_3C=O$), DPPA = diphenylphosphorylazide, TEA = triethylamine, EDC = N-ethyl-N′-3-dimethylaminopropylcarbodiimide hydrochloride, HOBT = 1-hydroxybenzotriazole, DMF = dimethyl formamide, TFA = trifluoroacetic acid, Ms = mesyl (methane sulfonyl), Bz = benzyl, Boc = t-butoxycarbonyl, THF = tetrahydrofuran, Im = imidazolyl, and DMAP = N,N-dimethylaminopyridine. All starting materials are either available from commercial sources, and/or are described in the literature (where cited).

Scheme 2 -

Step 1:

Cbz-Ala-COOH 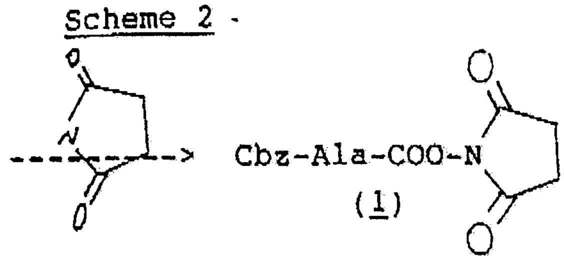

(1)

Step 2:

1 + Gly/EtOH/$NaHCO_3$ ---> Cbz-Ala-Gly

(2)

Step 3:

2 + DPPA/TEA/t-BuOH ---> Cbz-Ala-C(O)$NHCH_2$NH-Boc

(3)

Step 4:

3 + $H_2$/Pd, EtOH -----> $NH_2$-Ala-C(O)$NHCH_2$NH-Boc

(4)

Step 5:

4 + 1, EtOH -----> Cbz-Ala-Ala-C(O)$NHCH_2$NH-Boc

(5)

Step 6:

5 + $H_2$/Pd, DMF ---> $NH_2$-Ala-Ala-C(O)$NHCH_2$NH-Boc

(6)

Step 7:

$MeNHCH_2COOH$ + $HOCH_2(C_6H_5)$ --> $MeNHCH_2COOCH_2(C_6H_5)$

(7)

Step 8:

7 + AcOAc + TEA ---> $MeC(O)N(Me)CH_2COOCH_2(C_6H_5)$

(8)

Scheme 2 (Cont.)

Step 9:

8 + Lawesson's Rgnt --> $MeC(=S)N(Me)CH_2COOCH_2(C_6H_5)$

(9)

Step 10:

9 + $NaOH/MeOH/H_2O$ ---> $MeC(=S)N(Me)CH_2COOH$

(10)

Step 11:

6 + 10 + EDC/HOBT/DMF -->

---> $MeC(=S)N(Me)CH_2CO(Ala)_2NHCH_2NH$-Boc

(11)

Step 12:

11 + MeI/AcOH --->

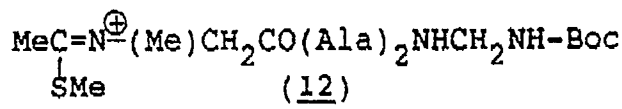

(12)

Step 13:

12 + TFA ----->

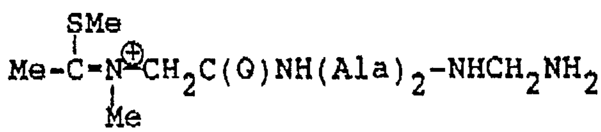

(13)

Step 14:

13 + weak base --->
ion exchange
resin/DMF

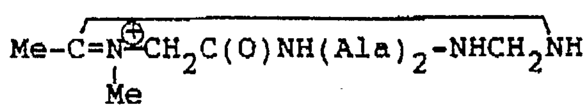

(14)

Using similar chemistry, a variant of a class 1 mimic (aminoethane amidinium link) was introduced in the context of replacing an (i+4)—→ hydrogen bond in one turn of an alpha helix as outlined in Scheme 3. Each step of the reaction scheme is set forth in detail in the Examples below. Due to the larger ring size (by one methylene group), the resulting structure exhibits a type 1 reverse turn conformation in DMSO and water, as demonstrated unequivocally by nmr experiments. Approximately 25% of native proteins exhibit reverse turn conformations.

Scheme 3

Step 1:

$NH_2CH_2CH_2NH_2$ + Cbz-Cl/MsOH/KOAc --> $Cbz\text{-}NHCH_2CH_2NH_2$ (15)

Step 2:

15 + $(t\text{-}BuO)_2CO$, EtOAc --> $Cbz\text{-}NHCH_2CH_2NH\text{-}Boc$ (16)

Step 3:

16 + $H_2$/Pd, EtOH ---> $NH_2CH_2CH_2NH\text{-}Boc$ (17)

Step 4:

17 + 1, EtOH ---> $Cbz\text{-}Ala\text{-}CONHCH_2CH_2NH\text{-}Boc$ (18)

Step 5:

18 + $H_2$/Pd, EtOH ---> $NH_2\text{-}Ala\text{-}CONHCH_2CH_2NH\text{-}Boc$ (19)

Step 6:

19 + 1, EtOH ---> $Cbz\text{-}(Ala)_2\text{-}CONHCH_2CH_2NH\text{-}Boc$ (20)

Step 7:

20 + $H_2$/Pd, EtOH ---> $NH_2\text{-}(Ala)_2\text{-}CONHCH_2CH_2NH\text{-}Boc$ (21)

Step 8:

$MeNHCH_2COOH$ + TsOH/BzOH/BzH --> $MeNHCH_2COOBz$ (22)

Step 9:

22 + TEA/$Ac_2O$/THF --->

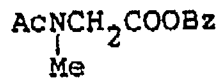

(23)

Step 10:

23 + Lawesson's Rgt --->

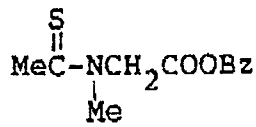

(24)

Step 11:

24 + NaOH/MeOH/$H_2O$ -->

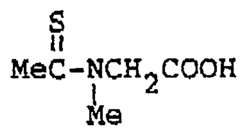

(25)

Step 12:

21 + 25 -->

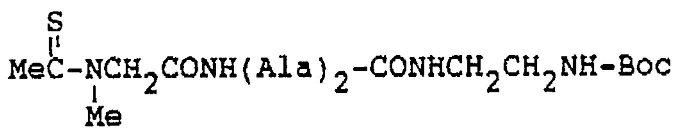

(26)

Step 13:

26 + MeI/AcOH -->

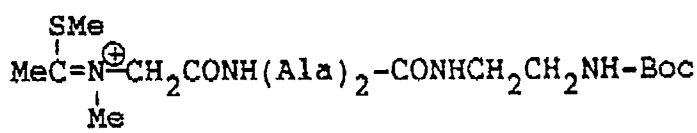

(27)

Step 14:

27 + TFA -->
EtOH

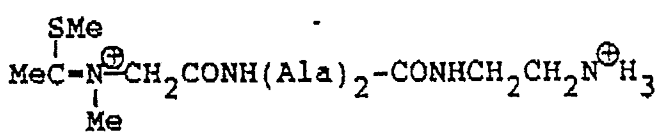

(28)

Step 15:

28 + TEA, EtOH -->

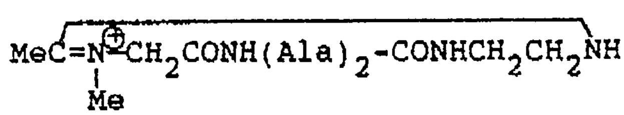

(29)

Synthesis of Class 2 Mimics

The class 2 mimics are generated by reaction of a hydrazine derivative with an acetal or ketal according to the general reaction:

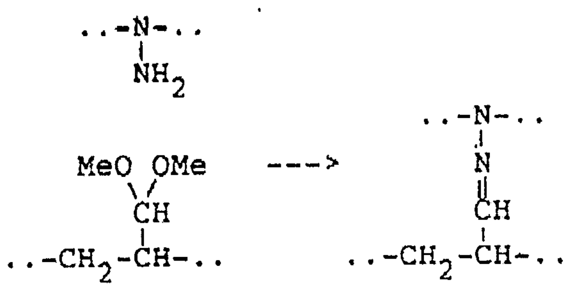

In the context of intrachain bonding, the reaction is as shown in Scheme 4.

In the case of a class 2 mimic, the hydrogen bond is replaced by the carbon-nitrogen double bond (C=N) of an N-acyl hydrazone. This hydrazone is formed by a condensation reaction between an N-amido peptide and a peptide fragment bearing an aldehyde moiety, masked in the form of an acetal. Scheme 4 outlines the synthesis of class 2-containing peptides. The synthesis is a convergent one. The two fragments 31 and 37 are synthesized in parallel, then combined in a peptide condensation reaction. In the final step, the hydrogen bond mimic is formed by a cyclo-condensation reaction between the hydrazide and the acetal functional groups. Three types of 31 fragment (31a, 31b, 31c) are illustrated.

Scheme 4

$(MeO)_2CH(CH_2)_3COOH$ + (1) $Im_2CO$; (2) $MeNHCH_2COOBz$ -->

-----------> $(MeO)_2CH(CH_2)_3CONCH_2COOBz$

Me

(30)

30 + $H_2$/Pd, MeOH --> $(MeO)_2CH(CH_2)_3CONCH_2COOH$

Me

(31a)

$(MeO)_2CH(CH_2)_3COOH$ + (1) $Im_2CO$; (2) $NH_2C(R)HCOOBz$ -->

-----------> $(MeO)_2CH(CH_2)_3CONHCHCOOBz$

R

(32)

R

32 + $(t\text{-}BuOC)_2O$, DMAP --> $(MeO)_2CH(CH_2)_3CONCHCOOBz$

Boc

(33)

R

33 + $H_2$/Pd, MeOH --> $(MeO)_2CH(CH_2)_3CONCHCOOH$

Boc

(31b)

R

32 + $H_2$/Pd, MeOH --> $(MeO)_2CH(CH_2)_3CONHCHCOOH$

(31c)

$H_2NNHCH_2COOEt$ + acetone --> $Me_2C{=}NNHCH_2COOEt$

(<u>34</u>)

<u>34</u> + $(Boc\text{-}NHCHR'CO)_2O$ --> $Boc\text{-}NHCH(R')\text{-}CON(NH_2)\text{-}CH_2COOEt$

(<u>35</u>)

<u>35</u> + Cbz-Cl --------> $Boc\text{-}NHCH(R')\text{-}CON(NH\text{-}Cbz)\text{-}CH_2COOEt$

(<u>36</u>)

<u>36</u> + HCl --------> $HCl\cdot H_2NCH(R')\text{-}CON(NH\text{-}Cbz)\text{-}CH_2COOEt$

(<u>37</u>)

<u>31</u> + <u>37</u> ---> $(MeO)_2CH(CH_2)_3CON(X)CH(R)CONHCH(R')\text{-}CON(NH\text{-}Cbz)\text{-}CH_2COOEt$

(<u>38</u>)

X = Me (<u>31a</u>)
Boc (<u>31b</u>)
H (<u>31c</u>)

<u>38</u> + $H_2$/Pd ---> $(MeO)_2CH(CH_2)_3CON(X)CH(R)CONHCH(R')\text{-}CON(NH_2)\text{-}CH_2COOEt$

(<u>39</u>)

39 + $BF_3 \cdot Et_2O$ --->

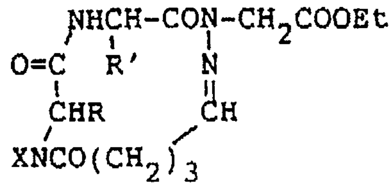

(40)

The synthesis of one particular peptide containing a class 2 hydrogen bond mimic is shown in Scheme 5. The experimental details are set forth in the Examples below. All syntheses were characterized by nmr spectroscopy and high resolution mass spectroscopy. Detailed conformational analysis of these peptides were carried out using a combination of one and two-dimensional nmr techniques, including Difference Nuclear Overhauser Effect spectroscopy, COSY spectroscopy, and coupling constant analysis. The product produced is helical in character.

Scheme 5

Step 1:

$(MeO)_2CH(CH_2)_3COOH$ + (1) $Im_2CO$ (2) $NH_2C(i\text{-}Bu)HCOOBz$ --> ----------> $(MeO)_2CH(CH_2)_3CONHCHCOOBz$

i-Bu

(32)

Step 2:

i-Bu

32 $H_2/Pd$, MeOH --> $(MeO)_2CH(CH_2)_3CONHCHCOOH$

(31c)

Step 3:

$H_2NNHCH_2COOEt$ + acetone --> $Me_2C{=}NNHCH_2COOEt$

(34)

Step 4:

34 + $(Boc\text{-}NHCHMeCO)_2O$ --> $Boc\text{-}NHCH\text{-}CON\text{-}CH_2COOEt$

Me $NH_2$

(35)

Step 5:

35 + Cbz-Cl --------> $Boc\text{-}NHCH\text{-}CON\text{-}CH_2COOEt$

Me NH-Cbz

(36)

Step 6:

36 + HCl -------> $HCl\cdot H_2NCH\text{-}CON\text{-}CH_2COOEt$

Me NH-Cbz

(37)

Step 7:

31c + 37 --->

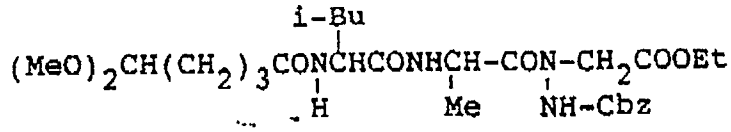

(38)

Step 8:

38 + $H_2Pd$ -->

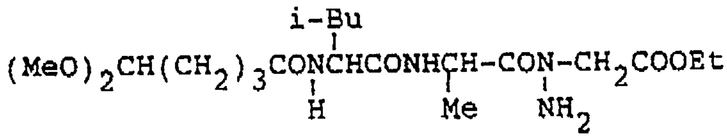

(39)

Step 9:

39 + $BF_3 \cdot Et_2O$ --->

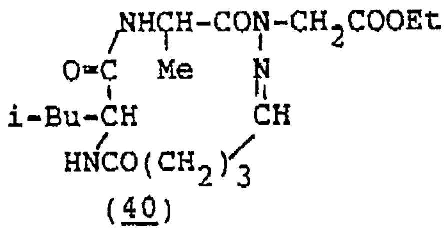

(40)

Thus, both classes of mimic can be used to stabilize a conformation ordinarily stabilized by hydrogen bonding in the context of a growing peptide chain, or can be formed even more simply between parallel or antiparallel chains in sheets. This evidences the broad scope of utility of these mimics, which can be put into the context of any desired peptide.

## Examples

The following Examples are presented as a further illustration of the practitioner of ordinary skill in the art, and are not intended to limit the scope of the invention in any manner. The following examples demonstrate solution-phase synthesis; however, other methods (e.g., solid phase synthesis) are also considered within the scope of this invention.

### Example 1

(Scheme 2, Step 1)

N-benzyloxycarbonyl-alanine N-hydroxysuccinimide ester (1) was synthesized according to the procedure described by G.W. Anderson et al, J Am Chem Soc, 86:1839 (1964).

Briefly, equal moles of N-Cbz-ala, N-hydroxysuccinimide, and dicyclohexylcarbodiimide were added in THF in an ice-water bath with stirring. The mixture was kept in a cool room (0°C) overnight. The dicyclohexylurea precipitate was removed by filtration, and the solvent evaporated in vacuo. The residue was crystallized from isopropanol to yield 1.

### Example 2

(Scheme 2, Step 2)

Cbz-alanine N-hydroxysuccinimide ester (1, 20 mM) was dissolved in absolute ethanol (EtOH, 50 ml), and the solution added to a solution of glycine (20 mM) and $NaHCO_3$ (40 mM) in water (50 ml). The mixture was stirred at room temperature overnight, concentrated to a small volume with a rotary vacuum evaporator, and

acidified to pH 2 with concentrated HCl. The product was crystallized by cooling the mixture in an ice-water bath, and recrystallized from ethyl acetate-hexane to provide Cbz-Ala-Gly (2).

## Example 3

(Scheme 2, Step 3)

Following the procedure of T. Shioiri et al, J Am Chem Soc, 94:17 (1972), equal moles of Cbz-Ala-Gly (2), diphenylphosphorylazide (DPPA), and triethylamine (TEA) were dissolved in t-butanol. The mixture was refluxed overnight. After evaporation of the solvent, the neutral fraction in ethyl acetate obtained after aqueous acid and alkali work ups was purified by silica gel column chromatography or crystallization from ethyl acetate-hexane, to yield Cbz-Ala-$CONHCH_2NH$-Boc (3).

## Example 4

(Scheme 2, Step 4)

To a solution of 3 (10 mM) in EtOH in a round bottom flask was added a small amount of Pd/C catalyst. A balloon filled with hydrogen was attached to the flask. After repeated degassing, the flask was filled with hydrogen and the mixture vigorously stirred for 30 minutes and filtered through celite to provide $NH_2$-Ala-$CONHCH_2$-Boc (4).

## Example 5

(Scheme 2, Step 5)

Cbz-alanine N-succinimide ester (1, 10 mM) was added to the 4 product solution from Example 4. The mixture was agitated overnight and concentrated to a small volume with a rotary vacuum evaporator. The product was crystallized from the mixture with addition of distilled water to yield Cbz-Ala-Ala-$CONHCH_2NH$-Boc (5).

## Example 6

(Scheme 2, Step 6)

Compound 5 was deprotected by catalytic hydrogenolysis using the procedure described in Example 4 above, to provide $NH_2$-Ala-Ala-$CONHCH_2NH$-Boc (6).

## Example 7

(Scheme 2, Step 7)

Sarcosine (250 mM) and p-toluenesulfonic acid (TsOH, 255 mM) were added to a mixture of benzyl alcohol (100 ml) and toluene (50 ml). The mixture was heated to reflux, and the water formed by the reaction trapped in a Dean Stark receiver. When no more water appeared in the distillate, the mixture was cooled to room temperature, added to ether, and cooled in an ice water bath for two hours. The crystalline product was collected on a filter and recrystallized from $CH_2Cl_2$-$Et_2O$ to yield $MeNHCH_2COOCH_2(C_6H_5)$ •TsOH (7).

Example 8

(Scheme 2, Step 8)

Acetic anhydride (AcOAc, 10 mM) was slowly added to a solution of 7 (10 mM) and TEA (20 mM) in THF with stirring. The mixture was left to stand at room temperature for two hours after the addition of acetic anhydride was completed. After the solvent was removed by vacuum evaporation the residue was redissolved in ethyl acetate and washed with citric acid (1 M), $NaHCO_3$ (0.5 M), water, saturated NaCl solution, and dried over anhydrous $MgSO_4$. The product was recovered by the removal of the solvent with a vacuum evaporator to yield $AcN(Me)CH_2COOCH_2(C_6H_5)$ (8).

Example 9

(Scheme 2, Step 9)

Following the procedure described by S. Scheibye et al, Bull Soc Chim Belg, 87:229 (1978), p-methoxyphenylthionophosphine sulfide (Lawesson's Reagent) was added to a solution of 8 in benzene, and the mixture heated to reflux for 10 minutes. After cooling, the mixture was washed with saturated $NaHCO_3$ solution, citric acid, water, saturated NaCl solution, and dried over anhydrous $MgSO_4$. After the solvent was removed by vacuum evaporation, the residue was crystallized from ethyl acetate-hexane to provide $MeC(=S)N(Me)CH_2COOCH_2(C_6H_5)$ (9).

Example 10

(Scheme 2, Step 10)

A solution of N-thioacetyl sarcosine benzylester (9, 10 mM) in methanol (20 ml) was surrounded by a water bath of room temperature, and 1 N NaOH (22 ml) was added with stirring. The mixture was left at room temperature for two hours, and treated with strongly acidic ion exchangers. After the solvent was removed by vacuum evaporation, the residue was crystallized from EtOAc-hexane to yield $MeC(=S)N(Me)CH_2COOH$ (10).

Example 11

(Scheme 2, Step 11)

An equimolar mixture of 6, 10, N-ethyl-N′-3-dimethylaminopropylcarbodiimide hydrochloride (EDC), and 1-hydroxybenzotriazole (HOBT) in dimethylformamide (DMF) were reacted at 0°C overnight. The mixture was treated with mixed ion exchangers and the solvent removed by vacuum evaporation. The residue was crystallized from ethanol and water to yield the condensation product $MeC(=S)N(Me)CH_2CO(Ala)_2NHCH_2NH$-Boc (11).

Example 12

(Scheme 2, Step 12)

Compound 11 was dissolved in acetic acid and a molar excess of iodomethane added. After the mixture was stirred for six hours the solvent, the unreacted MeI was removed with a vacuum rotary evaporator. It was important to use acid acetic as solvent to stabilize the product in this step. Other solvents, such as DMF or THF resulted in a considerable side product formation. The reaction yielded $MeC(-SMe)=N^{\oplus}(Me)CH_2CO(Ala)_2NHCH_2NH$-Boc (12).

## Example 13

(Scheme 2, Step 13)

Compound 12 was treated with trifluoroacetic acid (TFA) for 10 minutes, and the solvent removed by vacuum evaporation to provide

$$Me-\underset{SMe}{\underset{|}{C}}=N^{\oplus}(Me)CH_2CONH(Ala)_2-NHCH_2NH_2 \quad (\underline{13}).$$

## Example 14

(Scheme 2, Step 14)

Compound 13 was dissolved in DMF at a concentration less than 10 mM and treated with weakly basic ion exchangers. After the solvent was removed by vacuum evaporation the residue was purified with HPLC to yield

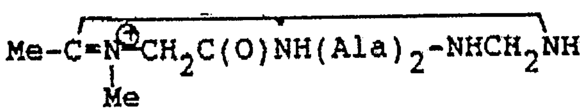 (14),

a compound of the invention.

## Example 15

(Scheme 3, Step 1)

Following the procedure described by G.J. Atwell et al, Synthesis, 1984:1032. Ethylene diamine (0.26 M) was dissolved in water containing bromocresol green as indicator. Methanesulfonic acid (0.48 M) in water (50 ml) was added until a blue to pale yellow color was achieved. The solution was diluted with EtOH (140 ml), vigorously stirred, and treated at room temperature with solutions of benzyloxycarbonyl chloride (Cbz-Cl, 0.23 M) in dimethoxyethane (DME, 50 ml) and 50% aqueous KOAc. After the additions were complete the mixture was stirred for one hour, and volatiles were removed in vacuo. The residue was shaken with water (500 ml) and filtered to remove the bis-derivative. The filtrate was washed with benzene, basified with 40% NaOH, and then extracted with benzene. The organic layer was washed with saturated aqueous NaCl, dried with $MgSO_4$, and dried in vacuo to yield Cbz-$NHCH_2CH_2NH_2$ (15).

## Example 16

(Scheme 3, Step 2)

Compound 15 was dissolved in ethyl acetate and an equimolar amount of t-butyl pyrocarbonate slowly added. The solution was dried with a vacuum rotary evaporator and the residue crystallized from ethyl acetate-hexane to yield Cbz-$NHCH_2CH_2NH$-Boc (16).

## Example 17

(Scheme 3, Step 3)

Following the procedure described in Example 4 above, compound 16 was hydrogenolyzed to provide $NH_2CH_2CH_2NH$-Boc (17).

Example 18

(Scheme 3, Step 4)

Following the procedure described in Example 5 above, compounds 17 and 1 were condensed in EtOH to provide Cbz-Ala-$CONHCH_2CH_2NH$-Boc (18).

Example 19

(Scheme 3, Step 5)

Following the procedure described in Example 4 above, compound 18 was hydrogenolyzed to provide $NH_2$-Ala-$CONHCH_2CH_2NH$-Boc (19).

Example 20

(Scheme 3, Step 6)

Following the procedure described in Example 5 above, compounds 19 and 1 were condensed in EtOH to provide Cbz-Ala-Ala-$CONHCH_2CH_2NH$-Boc (20).

Example 21

(Scheme 3, Step 7)

Following the procedure described in Example 4 above, compound 20 was hydrogenolyzed to provide $NH_2$-Ala-Ala-$CONHCH_2CH_2NH$-Boc (21).

Example 22

(Scheme 3, Step 8)

Following the procedure described in Example 7 above, $MeNHCH_2COOH$ is esterified with benzyl alcohol to provide $MeNHCH_2COOBz$ (22).

Example 23

(Scheme 3, Step 9)

Following the procedure described in Example 8 above, compound 22 is acetylated to provide

AcNH(Me)$CH_2COOBz$ (23).

Example 24

(Scheme 3, Step 10)

Following the procedure described in Example 9 above, compound 23 is converted to MeC(=S) N© CH$_2$COOBz (24).
Me

Example 25

(Scheme 3, Step 11)

Following the procedure described in Example 10 above, compound 24 is saponified to yield MeC(=S)N© (Me)$CH_2COOH$ (25).

Example 26

(Scheme 3, Step 12)

Following the procedure described in Example 11 above, compounds 21 and 25 are condensed to provide MeC(=S)N© (Me)$CH_2CONH(Ala)_2$-$CONHCH_2CH_2NH$-Boc 26).

Example 27

(Scheme 3, Step 13)

Following the procedure described in Example 12 above, compound 26is S-methylated with MeI to provide MeC(-SMe)=N© (Me)$CH_2CONH(Ala)_2$-$CONHCH_2CH_2NH$-Boc 27).

Example 28

(Scheme 3, Step 14

Following the procedure described in Example 14 above, compound 27 was deprotected by hydrolyzing the Boc group with TFA, thus providing
MeC(-SMe)=N© (Me)$CH_2CONH(Ala)_2$-$CONHCH_2CH_2N^{\oplus}H_3$ 28).

Example 29

(Scheme 3, Step 15

Following the procedure described in Example 13 above, compound 28 is cyclized with TEA to provide

$MeC{=}N^{\oplus}(Me){-}CH_2CONH(Ala)_2{-}CONHCH_2CH_2NH$, (29),

a compound of the invention. Compound 29 exhibits a reverse turn structure, as determined by nmr.

## Example 30

(Scheme 5, Step 1

12 mmoles methyl 5,5-dimethoxypentanoate (Stevens, R.V., et al, J Am Chem Soc, 7054, 1979) were dissolved in 20 ml 50% aqueous methanol. 16 mmoles NaOH were added and the mixture was stirred for 2 hr at room temperature. The solvent was then evaporated, the residue layered with 30 ml EtOAc, and the sodium salt neutralized with ice cold 1 N HCl. The aqueous phase was extracted with an additional 20 ml EtOAc, the organic extracts combined and dried over $K_2CO_3$. The product was filtered and the solvent evaporated to provide 5,5-dimethoxypentanoic acid.

Carbonyldiimidazole ($Im_2CO$, 10 mmoles) was added to a solution of 5,5-dimethoxypentanoic acid (10 mmole) in acetonitrile (13 ml), and the mixture stirred at room temperature for 40 minutes. Leucine benzyl ester tosylate (11 mmoles) was added, followed by diisopropylethylamine (11 mmoles), and the mixture stirred overnight at room temperature. The solvent was evaporated, and the residue dissolved in ethylacetate (50 ml), washed with ice cold 1 M citric acid (20 ml), 1 M $NaHCO_3$, (20 ml) and brine (20 ml). The product was dried over $MgSO_4$, the solvent evaporated, and the residue chromatographed on silica gel to provide $(MeO)_2CH(CH_2)_3CONHCH(i\text{-}Bu)COOBz$ (32).

## Example 31

(Scheme 5, Step 2)

Compound 32 (10 mmoles) was dissolved in 15 ml MeOH. 50 mg 10% Pd/C were added, and the mixture was hydrogenolyzed for 20 minutes at room temperature under 1 atm $H_2$ pressure. The mixture was then filtered and the solvent evaporated to provide
$(MeO)_2CH(CH_2)_3CONHCH(i\text{-}Bu)COOH$ (33).

## Example 32

(Scheme 5, Step 3)

N-aminoglycine ethylester•HCl (32 mmoles) was neutralized with $Na_2CO_3$ (32 mmoles), extracted with $CH_2Cl_2$, and dried over $K_2CO_3$. Then, acetone (10 ml) was added, and the $CH_2Cl_2$ evaporated. An additional 20 ml acetone was added, and the solvent evaporated. The residue was then dissolved in $CH_2Cl_2$ (20 ml), dried over $MgSO_4$, and the solvent evaporated to yield
$Me_2C{=}NNHCH_2COOEt$ (34).

## Example 33

(Scheme 5, Step 4)

Compound 34 was added to 1.1 equivalents of Boc-ala anhydride in THF and allowed to react for 12 hr. The crude product was dissolved in $CH_2Cl_2$, 1 N HCl added, and stirred for 2 hr. The phases were then separated, and the $CH_2Cl_2$ evaporated to obtain
$Boc\text{-}NHCH(Me)CON(NH_2)CH_2COOEt$ (35).

Example 34

(Scheme 5, Step 5)

Compound 35 (20 mmoles) in $Et_2O$ (20 ml), was treated with $NaHCO_3$ (25 moles), Cbz-Cl (25 mmoles), and 3 drops DMF in 5 ml $H_2O$. The mixture was stirred vigorously overnight. The, 1 ml pyridine was added to destroy excess Cbz-Cl. The $Et_2O$ phase was washed with 1 N HCl, 1 N $NaHCO_3$, and brine, dried, and the solvent evaporated. The residue was crystallized from $Et_2O$/hexane to provide Boc-NHCH(Me)CON(NH-Cbz)$CH_2$COOEt (36).

Example 35

(Scheme 5, Step 6)

The Boc protecting group was removed by reacting compound 36 with 4 N HCl/dioxane for 30 min at room temperature. The excess dioxane/HCl was then evaporated to provide $NH_2$CH(Me)CON(NH-Cbz)$CH_2$COOEt•HCl (37).

Example 36

(Scheme 5, Step 7)

Ethyl(dimethylaminopropyl)carbodiimide (8 mmoles) was added to an ice-cooled solution of compound 31 in $CH_3CN$ (15 ml), and the mixture stirred for 1 hr at 0°. Then, compound 37 (8 mmoles) was added, followed by diisopropylethylamine (8 mmoles) . The mixture was stirred overnight at 5° C, and the solvent evaporated. The residue was dissolved in EtOAc (30 ml), washed with ice cold 1 M citric acid (15 ml), 1 M $NaHCO_3$ (15 ml) and brine (15 ml), and dried overnight over $MgSO_4$. The solvent was evaporated, and the product chromatographed on silica gel to provide

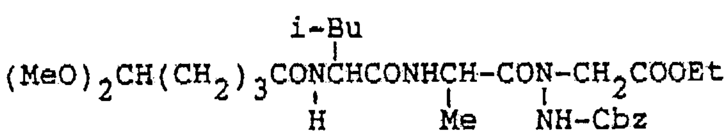 (38).

Example 37

(Scheme 5, Step 8)

Compound 38 (3 mmoles) in MeOH (15 ml) were hydrogenolyzed over 20 mg 10% Pd/C for 20 minutes at room temperature and under 1 atm $H_2$ pressure. The mixture was then filtered, and the solvent evaporated to provide

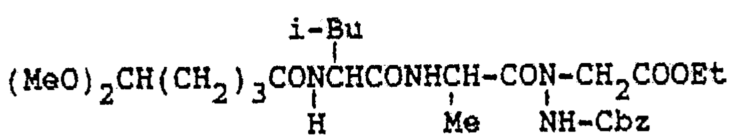 (39).

## Example 38

(Scheme 5, Step 9)

Compound 39 from Example 37 was dissolved in 100 ml $CH_3CN$. Then, $BF_3 \bullet Et_2O$ (10 ul) was added, and the solution stirred overnight at room temperature. The solvent was then evaporated, and the product chromatographed on a semi-preparative $C_4$ reverse phase HPLC column to provide the compound of the invention 40:

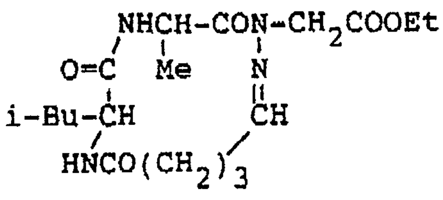

## Example 39

(Preparation of EGF Analogs)

A synthetic polypeptide having high epidermal growth factor activity is prepared as follows:

(A) Class 1 Mimic: A compound of the formula

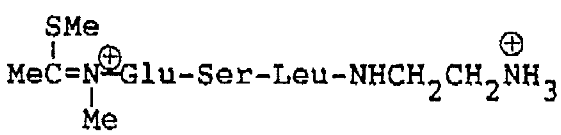

is prepared, following the procedures set forth in Examples 15-28. The compound is then cyclized following the procedure set forth in Example 29 to provide a reverse-turn stabilized EGF peptide of high activity.

(B) Class 2 Mimic: A compound of the formula $(MeO)_2CH(CH_2)_3CON$-Ile-Glu-Ser-Leu-Asp-Ser-Tyr-

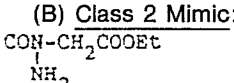

is prepared according to the procedures set forth in Examples 30-37, and is cyclized following the procedure of Example 38 to provide a small anti-parallel beta-sheet-reverse turn peptide, stabilized across the beta-sheet.

(C) Extended Class 2 Mimic: An extended class 2 EGF polypeptide analog is prepared similarly, by cyclizing a compound of the formula $(MeO)_2CH(CH_2)_3CON$-Met-His-Ile-Glu-Ser-Leu-Asp-Ser-Tyr-Thr-Cys-$CON(NH_2)$-$CH_2COOEt$.

## Example 40

(Preparation of Malarial Antigens)

A synthetic polypeptide vaccine for malaria was recently tested in humans by Herrington et al, Nature, 328:257 (1987). However, it proved to be only partially effective. Improvements in the potency of the vaccine could be achieved by the conformation restriction of the synthetic polypeptide, which is based upon a repeating tetrapeptide $(Asn\text{-}Ala\text{-}Asn\text{-}Pro)_3$. According to Chou-Fassman analysis (Chou et al, Biochem, 13:222 (1974)), the frame-shifted sequence Asn-Pro-Asn-Ala should have a strong tendency to form reverse turns in native proteins. The reverse turn can be locked into place by replacing putative amide-amide hydrogen bonds between Asn side chains around proline with class 1 or class 2 hydrogen bond mimics.

(A) Class 1 Mimic: A compound of the formula

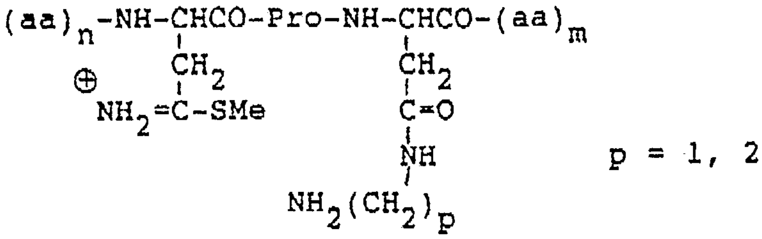

(where $(aa)_n$ and $(aa)_m$ are each independently amino acid sequences of 1-12 amino acids or R″, where R″ is alkyl of 1-6 carbons, phenyl, naphthyl, benzyl, or $-NH_2$) is prepared following the procedures set forth in Examples 1-29, and is cyclized to form a polypeptide analog suitable for malarial vaccination having the following structure:

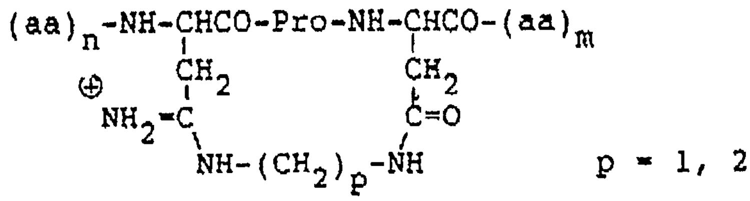

The compound is emulsified in a non-toxic vegetable oil and sterile water for injection, and is administered conventionally through intramuscular injection.

## Claims

1. A method for preparing active polypeptides based on active protein structures, which method comprises:
determining an approximate three-dimensional structure for the active region of said active protein;
identifying a stabilizing hydrogen bond; and
preparing a polypeptide having the same amino acid sequence as the active region of said active protein, wherein said stabilizing hydrogen bond is replaced by a bridging divalent radical selected from $-CH_2-NH-$, $-CH_2CH_2-NH-$, and -N=CH-.

2. A stabilized polypeptide, which comprises:
a sequence of at least three amino acids or amino acid equivalents linked by peptide bonds, wherein the hydrogen atom bound to one amide nitrogen (N) and the oxygen atom of a carbonyl (C) bond are replaced by a bridging divalent radical selected from
$-(N)-CH_2-NH-(C)-$, $-(N)-CH_2-CH_2-NH-(C)-$, and -(N)-N=CH-(C)-.

3. The polypeptide of claim 2 wherein said bridging divalent radical replaces a putative hydrogen bond.

4. The polypeptide of claim 3 wherein said bridging divalent radical links atoms forming a portion of the polypeptide backbone.

5. The polypeptide of claim 3 wherein said bridging divalent radical links an amino acid side chain with a second side chain or with the polypeptide backbone.

6. The polypeptide of claim 2 wherein said amino acid chain and said bridging diradical are selected to form a ring having 7, 10, 13, 14, 16, or 22 atoms.

7. A compound of the formula

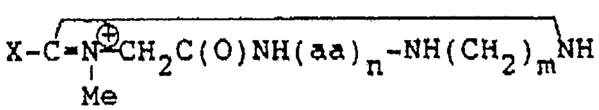

wherein,
$(aa)_n$ is an amino acid sequence of 1-12 amino acids;
m is an integer from 1-6; and
X is hydrogen, halo, alkyl of 1-6 carbons, acyl of 1-6 carbons, benzyl, haloalkyl of 1-6 carbons, or an amino acid sequence of 1-40 amino acids.

8. The compound of claim 7 wherein $(aa)_n$ is Ala-Ala, m is 1, and X is methyl.

9. The compound of claim 7 wherein $(aa)_n$ is Ala-Ala, m is 2, and X is methyl.

10. The compound of claim 7 wherein $(aa)_n$ is Glu-Ser-Leu, m is 2, and X is methyl.

11. A compound of the formula

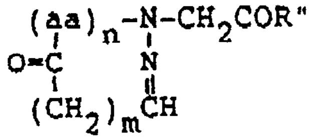

wherein,
R″ is alkoxy of 1-6 carbons, phenoxy, naphthyloxy, benzoxy, or $-NH_2$;
$(aa)_n$ is an amino acid sequence; and
m is an integer from 1-6.

12. The compound of claim 11 wherein $(aa)_n$ is Ile-Glu-Ser-Leu-Asp-Ser-Tyr, m is 3, and R″ is $NH_2$.

13. The compound of claim 11 wherein $(aa)_n$ is Leu-Ala, m is 3, and R″ is Et.

14. The compound of claim 11 wherein $(aa)_n$ is Met-His-Ile-Glu-Ser-Leu-Asp-Ser-Tyr-Thr-Cys, m is 3, and R″ is $-NH_2$.

15. A compound of the formula

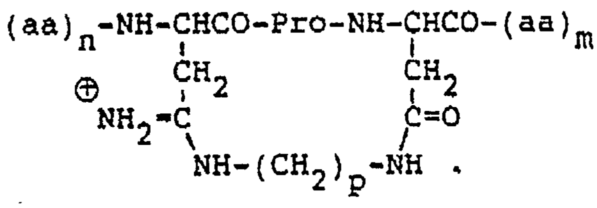

wherein,
$(aa)_n$ and $(aa)_m$ are each independently amino acid sequences of 1-12 amino acids or R″, where R″ is alkyl of 1-6 carbons, phenyl, naphthyl, benzyl, or $-NH_2$; and
p is 1 or 2.

16. The compound of claim 15 wherein $(aa)_n$ and $(aa)_m$ are each independently Ala or R″.

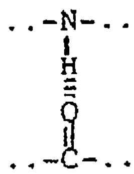

Normal

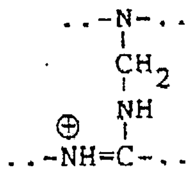

class 1

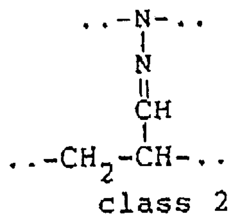

class 2

FIGURE 1